# EUROPEAN PATENT SPECIFICATION

(11) **EP 3 874 059 B1**
(45) Date of publication and mention of the grant of the patent: **20.08.2025**
(21) Application number: 19790567.2
(22) Date of filing: 29.10.2019
(51) Int. Cl.: C12Q 1/6806, C12N 15/10

(54) **SINGLE CELL FULL LENGTH RNA SEQUENCING**
VOLLLÄNGEN-RNA-SEQUENZIERUNG EINER EINZELZELLE
SÉQUENÇAGE MONOCELLULAIRE D'ARN PLEINE LONGUEUR

(30) Priority: 29.10.2018 EP 18203188
(43) Date of publication of application: 08.09.2021
(73) Proprietor: Koninklijke Nederlandse Akademie van Wetenschappen, 1011 JV Amsterdam (NL)
(72) Inventor: VAN OUDENAARDEN, Alexander, 3584 CT Utrecht (NL); SALMEN, Fredrik, 3584 CT Utrecht (NL)
(74) Representative: Nederlandsch Octrooibureau
(86) International application number: PCT/EP2019/079512
(87) International publication number: WO 2020/089218

(56) References cited:
- WO-A1-2017/048993
- WO-A1-2018/089550
- WO-A2-01/40458
- WO-A2-2005/064019
- US-A1- 2016 265 031
- ANDERS JEMT ET AL: "An automated approach to prepare tissue-derived spatially barcoded RNA-sequencing libraries", SCIENTIFIC REPORTS, vol. 6, no. 1, 16 November 2016 (2016-11-16), XP055518494, DOI: 10.1038/srep37137
- TAMAR HASHIMSHONY ET AL: "CEL-Seq: Single-Cell RNA-Seq by Multiplexed Linear Amplification", CELL REPORTS, vol. 2, no. 3, 27 September 2012 (2012-09-27), US, pages 666 - 673, XP055111758, ISSN: 2211-1247, DOI: 10.1016/j.celrep.2012.08.003
- R HRDLICKOVA ET AL: "RNA-Seq methods for transcriptome analysis.", WILEY INTERDISCIP REV RNA, vol. 8, no. 1, 19 May 2016 (2016-05-19), XP055652569
- ANNA M.L COENEN-STASS ET AL: "Evaluation of methodologies for microRNA biomarker detection by next generation sequencing", RNA BIOLOGY, vol. 15, no. 8, 18 September 2018 (2018-09-18), pages 1133 - 1145, XP055652572, ISSN: 1547-6286, DOI: 10.1080/15476286.2018.1514236

## Description

### Field of the invention

The present invention relates generally to the field of molecular biology. More particularly, it concerns methods for full length RNA sequencing of single-cells.

### Background of the invention

Single cell sequencing (SCS) has emerged as a powerful new tool for studying rare cells and delineating complex populations. The currently used methods are aimed at capturing the polyadenylated fraction of the transcriptome (~1% of the whole transcriptome). However, most single-cell protocols, such as Cel-Seq and Smart-seq (Hashimshony et al., Cell Reports 2(3):666-673 (2012), Hashimshony et al., Genome Biol. 17, 77 (2016) and Picelli, S. et al. Nat. Methods 10, 1096 (2013).) miss important RNA-species such as non-polyadenylated long non-coding RNA, tRNA, miRNA, snoRNA and snRNA. The key role of snoRNAs is to guide modifications of RNA, whilst most snRNAs are essential parts of the spliceosome. Hence, these RNA-species play a crucial function in RNAstructure and in the generation of different isoforms resulting in the translation of proteins with distinct functions.

Recently Hayashi et al (Nat. Commun. 9, (2018) described an SCS method, named RamDa-seq, for detecting nonpolyadenylated long non-coding RNA. However, this method does not allow to capture and simultaneously read out species of small RNAs in single-cells. Furthermore, it is not possible to tag the RNA molecules with barcodes and unique molecular identifiers (UMI), which makes it difficult to perform high-throughput sequencing and molecule counting using UMls.

Thus there remains a need for new methods that allow high throughput and full length RNA sequencing of single cells.

### Summary

The invention is defined by the scope of the appended claims. In particular, it relates to a method for preparing a sequencing library, preferably a deep-sequencing library, wherein the method comprises the steps of:
- step a) providing a sample containing RNA;
- step b) fragmenting the RNA;
- step c) end-repair to add an OH group at the 3' end of the fragmented RNA followed by polyadenylating the fragmented RNA;
- step d) hybridizing a poly-T primer to the polyadenylated RNA and performing reverse transcription of the hybridized RNA thereby obtaining cDNA;
- step e) performing a second strand synthesis;
- step f) in vitro transcription of the cDNA obtained in step d) thereby obtaining amplified RNA (aRNA);
- step g) ribosomal-RNA (rRNA) depletion; and
- step h) ligating an oligonucleotide adapter to the aRNA obtained in step f),
- step i) performing reverse transcription of the aRNA to obtain cDNA;
- step j) degrading the remaining aRNA;
wherein the poly-T primer comprises at least one of an identifier sequence (barcode) and a unique molecular identifier (UMI).

### Description of the invention

### Definitions

Unless defined otherwise, technical and scientific terms used herein have the same meaning as commonly understood by one of ordinary skill in the art to which this disclosure belongs. One skilled in the art will recognize many methods and materials similar or equivalent to those described herein, which could be used in the practice of the present invention. Indeed, the present invention is in no way limited to the methods and materials described.

For purposes of the present invention, the following terms are defined below.

Methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al. Molecular Cloning. A Laboratory Manual, 2nd Edition, Cold Spring Harbor Laboratory Press, Cold Spring Harbor, N. Y., 1989; Ausubel et al.. Current Protocols in Molecular Biology, John Wiley & Sons, New York, 1987 and periodic updates; and the series Methods in Enzymology, Academic Press, San Diego.

"A," "an," and "the": these singular form terms include plural referents unless the content clearly dictates otherwise. Thus, for example, reference to "a cell" includes a combination of two or more cells, and the like.

As used herein, the term "about" is used to describe and account for small variations. For example, the term can refer to less than or equal to ±10%, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%. Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range is explicitly specified. For example, a ratio in the range of about 1 to about 200 should be understood to include the explicitly recited limits of about 1 and about 200, but also to include individual ratios such as about 2, about 3, and about 4, and sub-ranges such as about 10 to about 50, about 20 to about 100, and so forth.

"And/or": the term "and/or" refers to a situation wherein one or more of the stated cases may occur, alone or in combination with at least one of the stated cases, up to with all of the stated cases.

"Comprising": this term is construed as being inclusive and open ended, and not exclusive. Specifically, the term and variations thereof mean the specified features, steps or components are included. These terms are not to be interpreted to exclude the presence of other features, steps or components.

"Primer based amplification" refers to a polynucleotide amplification reaction, namely, a population of polynucleotides that are replicated from one or more starting sequences, i.e. a primer. A suitable primer may have a sequence length of 15-30 nucleotides. Amplifying may refer to a variety of amplification reactions, including but not limited to polymerase chain reaction (PCR), linear polymerase reactions, nucleic acid sequence- based amplification, rolling circle amplification and the like.

"Sequencing" refers to determining the order of nucleotides (base sequences) in a nucleic acid sample.

"High throughput sequencing technologies, also referred to in the art as next generation sequencing, such as offered by Roche, Illumina and Applied Biosystems, or also referred to in the art as third generation sequencing, as described by David J Munroe & Timothy J R Harris in Nature Biotechnology 28, 426-428 (2010) and such as offered by Pacific Biosciences and Oxford Nanopore Technologies, may also be used. Such technologies allow from one sample DNA multiple sequence reads in a single run. For example, the number of sequence reads may range from several hundred up to billions of reads in a single run of a high through put sequence technology. High throughput sequencing technologies may be performed according to the manufacturer's instructions (as e.g. provided by Roche, Illumina or Applied Biosystems). The technology may involve the preparation of DNA before carrying out a sequencing run. Such preparation may include ligation of adaptors to DNA. Adaptors may include identifier sequences to distinguish between samples. Depending on the size of DNA that is suitable or compatible with the high throughput sequencing technology used, the DNA that is to be sequenced may be subjected to a fragmenting step.

"Size selection' according to the invention involves techniques with which particular size ranges of molecules, e.g. (ligated) DNA fragments or amplified (ligated) DNA fragments, are selected. Techniques that can be used are for instance gel electrophoresis, size exclusion, gel extraction chromatography, but are not limited thereto, as long as molecules with a particular size can be selected or excluded, such a technique will suffice.

The term "PCR" encompasses derivative forms of the reaction, including but not limited to, RT-PCR, real-time PCR, nested PCR, quantitative PCR, multiplexed PCR, assembly PCR and the like. Reaction volumes range from a few hundred nanoliters, e.g., 200 nL, to a few hundred microliters, e.g., 200 microliters. "Reverse transcription PCR," or "RT-PCR," means a PCR that is preceded by a reverse transcription reaction that converts a target RNA to a complementary single stranded DNA, which is then amplified, e.g., Tecott et al., U.S. Pat. No. 5,168,038. "Real-time PCR" means a PCR for which the amount of reaction product, i.e., amplicon, is monitored as the reaction proceeds. There are many forms of real-time PCR that differ mainly in the detection chemistries used for monitoring the reaction product, e.g., Gelfand et al., U.S. Pat. No. 5,210,015 ("Taqman"); Wittwer et al., U.S. Pat. Nos. 6,174,670 and 6,569,627 (intercalating dyes); Tyagi et al., U.S. Pat. No. 5,925,517 (molecular beacons). Detection chemistries for real-time PCR are reviewed in Mackay et al., Nucleic Acids Research, 30:1292-1305 (2002). "Nested PCR" means a two-stage PCR wherein the amplicon of a first PCR becomes the sample for a second PCR using a new set of primers, at least one of which binds to an interior location of the first amplicon. As used herein, "initial primers" in reference to a nested amplification reaction mean the primers used to generate a first amplicon, and "secondary primers" mean the one or more primers used to generate a second, or nested, amplicon. "Multiplexed PCR" means a PCR wherein multiple target sequences (or a single target sequence and one or more reference sequences) are simultaneously carried out in the same reaction mixture, e.g. Bernard et al. (1999) Anal. Biochem., 273:221-228 (two-color real-time PCR). Usually, distinct sets of primers are employed for each sequence being amplified. "Quantitative PCR" means a PCR designed to measure the abundance of one or more specific target sequences in a sample or specimen. Techniques for quantitative PCR are well-known to those of ordinary skill in the art, as exemplified in the following references: Freeman et al., Biotechniques, 26:112-126 (1999); Becker-Andre et al., Nucleic Acids Research, 17:9437-9447 (1989); Zimmerman et al., Biotechniques, 21:268-279 (1996); Diviacco et al., Gene, 122:3013-3020 (1992); Becker-Andre et al., Nucleic Acids Research, 17:9437-9446 (1989); and the like.

As used herein, the term "adapter" is a single-stranded, double-stranded, partly double-stranded, Y-shaped or hairpin nucleic acid molecule that can be attached, preferably ligated, to the end of other nucleic acids, and preferably has a limited length, e.g., about 10 to about 200, or about 10 to about 100 bases, or about 10 to about 80, or about 10 to about 50, or about 10 to about 30 bases in length, and is preferably chemically synthesized. The optionally double-stranded structure of the adapter may be formed by two distinct oligonucleotide molecules that are base paired with one another, or by a hairpin structure of a single oligonucleotide strand.

### Detailed description of the invention

As demonstrated herein we have developed Vast transcriptome Analysis in Single cells by A-tailing" (VASA-Seq), a novel single-cell method for whole transcriptome and full-length analysis. This method can be used for example, for single cells after FACS sorting, enabling multicolor antibody labeling of cell types (such as HSPCs) and recording of FACS-index data. VASA-Seq detects full-length isoforms of mRNA and long noncoding RNA with reduced technical noise due to the addition of UMIs to each fragment. It also provides strand information and, most importantly, is able to capture snoRNA and snRNA from the same cell. The method described herein allows better understanding of cell-to-cell heterogeneity due to broader detection of different RNA species in single cells.

The novelty is to perform fragmentation, end repair and poly-A tailing directly at the single cell level. This way, each fragment (and RNA species that naturally lack poly-A tails such as IncRNA, snRNA and snoRNA) can be primed with a barcoded poly-T primer. In some embodiments, the poly-T primer has a barcode and an UMI (unique molecular identifier) and it is thus possible to achieve much higher throughput in terms of the number of cells that can be processes compared to RamDa-seq (Hayashi et al, *supra*). RamDa-seq also lacks the UMI (can only be added in combination with a barcode) which is very important for noise reduction of the sequencing data. VASA-seq also exhibits strand specificity due to the fact that it's always the same end of the RNA fragments that gets poly-A tailed and primed. This feature is lacking in RamDa-seq or Smart-seq2 (an method for full-length RNA-sequencing of single cells, not full transcriptomics).

Thus, the methods described herein are suitable for generating NGS libraries corresponding to any RNA starting material of interest and are not limited to polyadenylated RNAs. For example, the subject methods may be used to generate NGS libraries from non-polyadenylated RNAs, including microRNAs, small RNAs, siRNAs, and/or any other type non-polyadenylated RNAs of interest. The methods also find use in generating strand-specific information, which can be helpful in determining allele specific expression or in distinguishing overlapping transcripts in the genome.

Disclosed is a method for processing an RNA sample, the method comprising the steps of:
- step a) providing a sample containing RNA;
- step b) optionally fragmenting the RNA;
- step c) (poly) adenylating the, optionally fragmented, RNA;
- step d) hybridizing a poly-T primer to the polyadenylated RNA and performing reverse transcription of the hybridized RNA thereby obtaining cDNA; and optionally
- step e) performing a second strand synthesis,
wherein the poly-T primer comprises at least one of an identifier sequence (barcode) and a unique molecular identifier (UMI).

In one embodiment, the method provides for producing a cDNA comprising a an identifier sequence (barcode) and a unique molecular identifier (UMI) the method comprising the steps:
- step a) providing a sample containing RNA;
- step b) optionally fragmenting the RNA;
- step c) (poly) adenylating the fragmented RNA;
- step d) hybridizing a poly-T primer to the polyadenylated RNA and performing reverse transcription of the hybridized RNA thereby obtaining cDNA; and optionally
- step e) performing a second strand synthesis,
wherein the poly-T primer comprises at least one of an identifier sequence (barcode) and a unique molecular identifier (UMI).

After the fragmentation step, the sample mixture will contain polyadynelated RNA and non-polyadenylated RNAs. Polyadenylation is the addition of a poly(A) tail to the fragmented RNA. It is an objective of the invention to polyadenylate the non-polyadenylated RNA so that these RNAs can sequenced. Adenylating of RNA may be performed using any convenient approach. According to certain embodiments, the adenylation is performed enzymatically, e.g., using Poly(A) polymerase or any other enzyme suitable for catalyzing the incorporation of adenine residues at the 3' terminus of the precursor RNA. Reaction mixtures for carrying out the adenylation reaction may include any useful components, including but not limited to, a polymerase, a buffer (e.g., a Tris-HCL buffer), one or more metal cations (e.g.. MgCb, MnCl2, or combinations thereof), a salt (e.g., NaCl), one or more enzyme-stabilizing components (e.g., DTT), ATP, and any other reaction components useful for facilitating the adenylation of a precursor RNA. The adenylation reaction may be carried out at a temperature (e.g., 30°C-50°C, such as 37°C) and pH (e.g., pH 7 - pH 8.5, such as pH 7.9) compatible with the polymerase being employed, e.g., polyA polymerase. Other approaches for adding nucleotides to a precursor RNA include ligation-based strategies, where an RNA ligase (e.g., T4 RNA ligase) catalyzes the covalent joining of a defined sequence to an end (e.g., the 3' end) of the precursor RNA to produce a template RNA.

In one embodiment the nucleotide sequence that is capable of hybridizing to nucleic acids is designed to hybridize to the poly-A tail of mRNA such as a poly T sequence. In one embodiment, the poly-T sequence and/or analogues thereof or a combination thereof comprise at least 6 nucleotides, 7, 8, 9, 10, 11, 12, 13, 14, 15, 16, 17, 18, 19, 20, 30 or 40 nucleotides.

As used herein, a "barcode" refers to a nucleic acid sequence that is preferably used to identify the cell or batch origin of nucleic acid after amplification and sequencing processes. The unique barcode sequence allows each cell's or batch's nucleic acids (genome or transcriptome) to be associated with the original cell/batch. In another embodiment the barcode sequence is used to trace back the genome to each cell. According to one embodiment, the barcode sequence comprises at least 2 nucleotides or alternatively, more than 2 nucleotides, or alternatively, at least 4 nucleotides, or alternatively, at least 6 nucleotides, or alternatively, at least 8 nucleotides, or alternatively, at least 10 nucleotides, or alternatively, at least 12 nucleotides, or alternatively, at least 14 nucleotides, or alternatively, at least 20 nucleotides, or alternatively, at most 8 nucleotides, or alternatively, more than 8 nucleotides, or alternatively, at most 10 nucleotides, or alternatively, at most 14 nucleotides, or alternatively, at most 20 nucleotides. In some embodiments, first strand synthesis is primed with an (anchored) oligo dT primer (or potentially with a randomer or a combination of the two) that is appended with a barcode, an amplification primer binding site, and optionally a template switch (TS) primer sequence.

In addition or alternatively, the prepared sequencing library and/or the processed RNA sample may comprise an UMI. Hence, the prepared sequencing library and/or the processed RNA may comprise at least one of a barcode and an UMI. In an embodiment, the barcode can be preceded or followed by a second barcode that is a molecular barcode (unique molecular identifier, or "UMI") that would allow for the detection of PCR duplicates. UMI sequences have been described in the art, such as by Kivioja et al., 2012, Nat Methods 9: 72-74. The UMI sequence is a random sequence which may be added to quantify absolute numbers of each transcript molecule and eliminate amplification biases. Thus, in some embodiments, 1st strand synthesis as performed in step d) is primed with an oligo dT that has been appended with a barcode, and a (UMI). It will be appreciated that the order of the barcode and UMI on the first strand synthesis primer can be varied. For example, in some embodiments, the barcode (BC) is positioned 3' to the UMI. In some embodiments, the barcode is positioned 5' to the UMI. In some embodiments, the barcode is directly contiguous with the UMI. In some embodiments, the barcode is separated from the UMI by 1, 2, 3, 4, 5, 6, 7, 8, 9, 10 or more than 10 nucleotides. In some embodiments, the sample barcode overlaps with the UMI by 1, 2, 3, 4, 5, 6, 7, 8, 9, 10 or more than 10 nucleotides.

In one embodiment of the invention, the RNA sample is a cellular RNA sample, preferably from a single cell, more preferably wherein the RNA is isolated from a cell nucleus. The invention is not limited to any specific cell type. Preferably, the cell is a nucleated cell. Preferably, the cell is a mammalian cell, preferably a human cell. A preferred human cell can be at least one of a tumor cell, an embryonic cell and a brain cell.

In an embodiment, the RNA sample comprises non-polyadenylated RNA that can become polyadenylated in the method of the invention. Preferably, the non-polyadenylated RNA that becomes polyadenylated, and optionally sequenced, in the method of the invention is a non-coding RNA. Preferably, the non-coding RNA is selected from the group consisting of long non-coding RNA (IncRNA), tRNA, miRNA, snoRNA and snRNA. Preferably, the non-coding RNA is a small non-coding RNA, preferably at least one of a miRNA, a snoRNA and a snRNA.

In one embodiment, the RNA sample is a cellular RNA sample, preferably the RNA sample is the total RNA of a single cell. In another one embodiment the RNA is isolated from a cell nucleus, preferably the RNA is isolated from a single cell nucleus, most preferably from a single cell nucleus.

As used herein, a "single cell" refers to one cell. Single cells useful in the methods described herein can be obtained from a tissue of interest, or from a biopsy, blood sample, or cell culture. Additionally, cells from specific organs, tissues, tumors, neoplasms, or the like can be obtained and used in the methods described herein. Furthermore, in general, cells from any population can be used in the methods, such as a population of prokaryotic or eukaryotic single celled organisms including bacteria, fungi or yeast.

In some embodiments of the method described herein, obtaining the RNA sample can include the step of first obtaining single cells and then lysing the cells to release the RNA.

A single cell suspension can be obtained using standard methods known in the art including, for example, enzymatically using trypsin or papain to digest proteins connecting cells in tissue samples or releasing adherent cells in culture, or mechanically separating cells in a sample. Single cells can be placed in any suitable reaction vessel in which single cells can be treated individually. For example a 96-well plate, such that each single cell is placed in a single well. Methods for manipulating single cells are known in the art and include fluorescence activated cell sorting (FACS), micromanipulation and the use of semi-automated cell pickers (e.g. the Quixell^{™} cell transfer system from Stoelting Co.). Individual cells can, for example, be individually selected based on features detectable by microscopic observation, such as location, morphology, or reporter gene expression.

Methods of lysis the cells or single cell to release the RNA are well known in the art. Lysis can be achieved by, for example, heating the cells, or by the use of detergents or other chemical methods, or by a combination of these. However, any suitable lysis method known in the art can be used. Preferably, the lysis step is performed by at least one of
- heating the cells; and
- proteinase K digestion in combination with a detergent, preferably in combination with the detergent IGEPAL.

In one embodiment of the invention, the fragmentation step b) is performed by a divalent metal -cation at a temperature between about 55-100°C.

Methods to fragment RNA are known to the skilled person. Common fragmentation methods include enzymatic fragmentation, nebulization, RNA hydrolysis and heat digestion of the RNA with a divalent metal cation, i.e. exposure to divalent cations at elevated temperature. In one embodiment of the invention, the fragmentation of RNA in step b) heated digestion of the RNA with a divalent metal cation. Preferably, the heat digestion is performed at a temperature between about 55-100°C, more preferably the heat digestion is performed at a temperature between about 65-85°C. In another embodiment of the invention the divalent metal cation is selected from the group consisting of Mg²⁺, Mn^{2+,} Ca ²⁺ and Zn ²⁺. Preferably, the divalent metal cation is Mg²⁺ or Mn²⁺.

In another embodiment of the invention, fragmentation of RNA in step b) can be carried out chemical reactions including for example, hydrolysis reactions including base and acid hydrolysis.

For example, in particular embodiments, RNA can be fragmented by Alkaline conditions because RNA is unstable under alkaline conditions. See, e.g., Nordhoff et al. (1993) "Ion stability of nucleic acids in infrared matrix-assisted laser desorption/ionization mass spectrometry", Nucl. Acids Res., 21(15):3347-57. In one embodiment of the invention, RNA is fragmented by hydrolysis using Na₂CO₃.

Fragmentation by the RNA can result in a phosphate group at the 3'end of the fragmented RNA. In one embodiment, end-repairing of the fragmented RNA might be required before polyadenylation can take place. The end-repairing step replaces the phosphate group, that is created by RNA fragmentation at the 3'end of the RNA strand, with an OH group so that the fragmented RNA can be subjected to polyadenylation. End-repair can be performed by methods known in the art, including for example, by using a polynucleotide kinase, preferably the T4 Polynucleotide Kinase (PNK) and a source of phosphate such as ATP.

In one embodiment, the method of the invention further comprises sequencing of the cDNA obtained in the claimed method.

In one embodiment next generation sequencing (NSG) is used, such as offered by Roche, Illumina and Applied Biosystems, or also referred to in the art as third generation sequencing, as described by David J Munroe & Timothy J R Harris in Nature Biotechnology 28, 426-428 (2010) and such as offered by Pacific Biosciences and Oxford Nanopore Technologies, may also be used.

The method of the invention comprises the following steps:
- step f) *in vitro* transcription of the cDNA obtained in step d) thereby obtaining amplified RNA (aRNA) and
- step g) depleting the ribosomal RNA (rRNA)

As used herein, *"in vitro transcription"* or "IVT" refers to the process whereby transcription occurs in vitro in a non-cellular system to produce "synthetic RNA molecules". In one embodiment, the method of the invention comprises in vitro transcription (step f) of the cDNA obtained in step d), whereby amplified RNA (aRNA) is obtained. By "amplified RNA" it is meant that for each initial source of nucleic acid, multiple corresponding RNAs are produced.

The person skilled in the art straightforwardly understands that that there can be additional or alternative methods to amplify the cDNA obtained in step d), such as, but not limited to polymerase chain reaction (PCR). As a non-limiting example, PCR may be used instead of, or in addition to, IVT when *e.g*. an adapter is ligated at the 5' site of the, optionally poly-adenylated, RNA of step a), b) or c) and/or an adapter may be ligated to the cDNA obtained in step d). This adapter may comprise a primer binding site for amplifying the cDNA. A second primer binding site may be located in the poly-T primer and or in any further adapter ligated to the other site of the cDNA molecule, such that the cDNA molecule is flanked by primer binding sites for amplifying the cDNA.

Ribosmal RNA (rRNA) comprises about 95% to about 98% of the RNA in a cell, its presence can complicate the analyses of RNA molecules of interest in a sample. In one embodiment, the method of the invention thus includes a step of rRNA depletion. The skilled person straightforwardly understands that the cDNA molecule obtained in step d) of the method of the invention can be further extended with any preferred additional nucleotide sequence, preferably to extend the cDNA molecule of step d) with one or more universal sequences as specified herein. Preferably, the cDNA molecule in the sequencing library comprises on one site of the molecule at least an UMI obtained by reverse transcription as specified in step d), and at the other site any preferred additional nucleotide sequence, such as, but not limited to, one or more universal sequences, a barcode and/or an UMI.

For example, at different steps of the method as specified herein, there may be one or more adapters ligated to the cDNA and/or RNA molecule. In addition or alternatively, additional nucleotide sequences may be added during the reverse transcription and/or amplification steps by incorporating these sequences in the primer used for respectively reverse transcription and/or amplification.

The method of the invention comprises a step h) of ligating an oligonucleotide adapter to the aRNA obtained is step f). Preferably, the adapter-ligated RNA molecule comprises at one site of the molecule at least an UMI obtained by reverse transcription as specified in step d), and at the other site an adapter obtained in step h). Preferably, the adapter comprises one or more universal sequences as defined herein. The adapter may further comprise at least one of a barcode and an UMI.

Alternatively or in addition, the adapter can be ligated to at least one of:
- the RNA provided in the sample of step a);
- the single-stranded cDNA obtained in step d);
- the double-stranded cDNA obtained in step e); and
- the cDNA obtained in step i).

In an embodiment, the adapter is ligated to the RNA provided in step a). Preferably, the adapter is ligated to the 5' end of the RNA molecule. Preferably, the adapter comprises one or more universal sequences as defined herein. The adapter may further comprise at least one of a barcode and an UMI. Optionally, the 5' end is phosphorylated prior to adapter ligation.

In an embodiment, the adapter is ligated to the cDNA obtained in step d). Preferably, the adapter-ligated cDNA comprises on one site of the molecule an UMI obtained by reverse transcription as specified in step d), and comprises at the other site an adapter. Preferably, the adapter comprises one or more universal sequences as defined herein. The adapter may further comprise at least one of a barcode and an UMI.

In an embodiment, the adapter is ligated to the double-stranded cDNA obtained in step e). Preferably, the adapter-ligated double-stranded cDNA molecule comprises at one site of the molecule an UMI obtained by reverse transcription as specified in step d), and at the other site an adapter. Preferably, the adapter comprises one or more universal sequences as defined herein. The adapter may further comprise at least one of a barcode and an UMI.

The method of the invention comprises a step i) of performing reverse transcription of the aRNA. In an embodiment, the adapter is ligated to the cDNA molecule obtained in step i). Preferably, the adapter-ligated cDNA comprises on one site of the molecule an UMI obtained by reverse transcription as specified in step d), and comprises at the other site an adapter. Preferably, the adapter comprises one or more universal sequences as defined herein. The adapter may further comprise at least one of a barcode and an UMI.

In an embodiment, any preferred additional sequence, preferably any universal sequence, is present in a primer used in step e) to perform the second-synthesis. The primer may further comprise at least one of a barcode and an UMI.

In an embodiment, any preferred additional sequence, preferably any universal sequence, is present in a primer used in step i) to perform reverse transcription. The primer may further comprise at least one of a barcode and an UMI.

In an embodiment, any preferred additional sequence, preferably any universal sequence, is present in a primer used in step k) to amplify the cDNA. The adapter may further comprise a barcode.

The method of the invention comprises a step j) of degrading the remaining aRNA.

In an embodiment, the method of the invention further comprises a step k) of amplifying the cDNA samples to generate a cDNA library comprising double- stranded cDNA.

In an embodiment, the method of the invention further comprises a step l) of selecting by size the cDNA library obtained in step k).

In an embodiment, the method of the invention further comprises a step m) of sequencing at least one of:
- the cDNA library generated in step k); and
- the size selected cDNA library from step l).

In one embodiment, the method of the invention comprises the following steps:
- step h) ligating an oligonucleotide adapter to the aRNA obtained is step f;
- step i) performing reverse transcription of the adapter-ligated aRNA;
- step j) degrading the remaining aRNA;
- step k) optionally amplifying the cDNA samples to generate a cDNA library comprising double- stranded cDNA;
- step I) selecting by size the amplified PCR product obtained in step k); and
- step m) sequencing of the size selected PCR products from step l).

The adapters that are added to the 5' and/or 3' end of a nucleic acid can comprise a universal sequence.

In an embodiment, the cDNA molecules of the sequencing library, e.g. the cDNA molecules obtained by the method of the invention, are flanked by one or more universal sequences.

A universal sequence is a region of nucleotide sequence that is common to, i.e., shared by, two or more nucleic acid molecules. Optionally, the two or more nucleic acid molecules also have regions of sequence differences. Thus, for example, the 5' adapters can comprise identical or universal nucleic acid sequences and the 3' adapters can comprise identical or universal sequences. A universal sequence that may be present in different members of a plurality of nucleic acid molecules can allow the replication or amplification of multiple different sequences using a single universal primer that is complementary to the universal sequence. Some universal primer sequences used in examples presented herein include the V2.A14 and V2.B15 sequences. However, it will be readily appreciated that any suitable adapter sequence can be utilized in the methods presented herein. The universal sequences may comprise a binding site for a sequencing primer, preferably a binding site for a deep-sequencing primer.

Methods to degrade the remaining a RNA are known in the art and include for example, degrading the RNA with a DNAase-free RNAse, such as RNaseA.

Optional step k) is carried out to prepare a standard NGS library, including ligating sequencing adapters to the DNA templates for direct sequencing.

In one embodiment of the invention, the size of the size selected PCR products is between 150bp and 1000bp, preferably the size of the selected PCR products is between 300-450.

The cDNA fragment comprising a barcode and a UMI obtainable by the methods according to the invention may be further processed to be sequenced either by sanger sequencing of by NSG sequencing as described above.

### Description of the figures

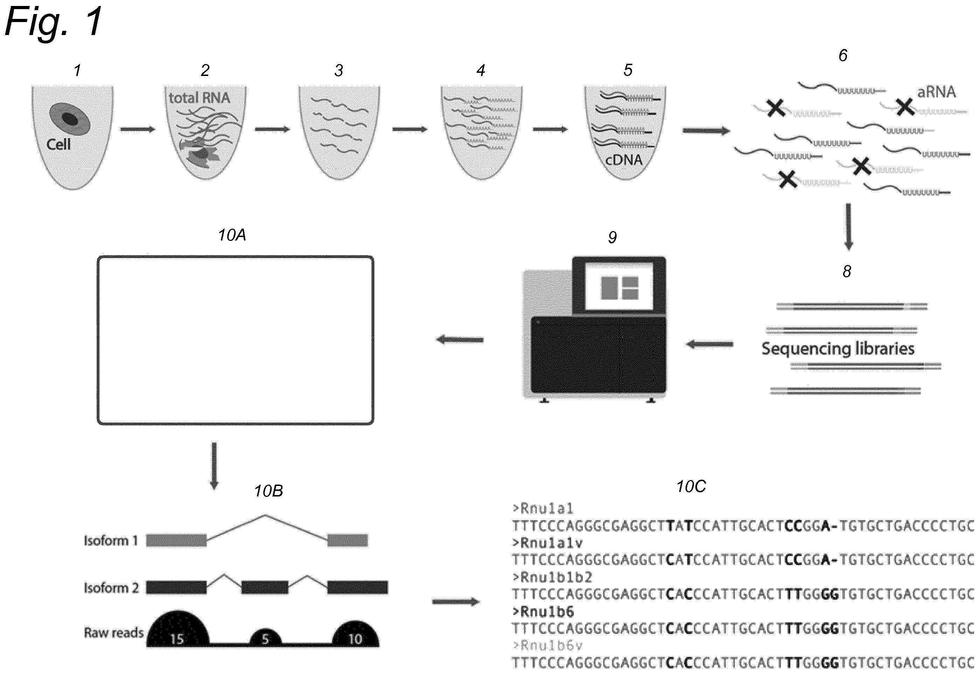

**Figure 1****:** Vast transcriptome Analysis in Single cells by A-tailing (VASA-seq). Schematic representation of the method according to one embodiment of the invention. 1) Cell sorting, 2) Cell lysis, 3) RNA fragmentation, 4) A-tailing, 5) Reverse transcription (Barcode + UMI), 6) IVT and rRNA depletion, 8) Modified Cel-seq/Cel-seq2 protocol (sequencing libraries), 9) Sequencing, 10A) Alignment and gene counting, 10B) Isoform quantification and 10C) snRNA variant analysis.

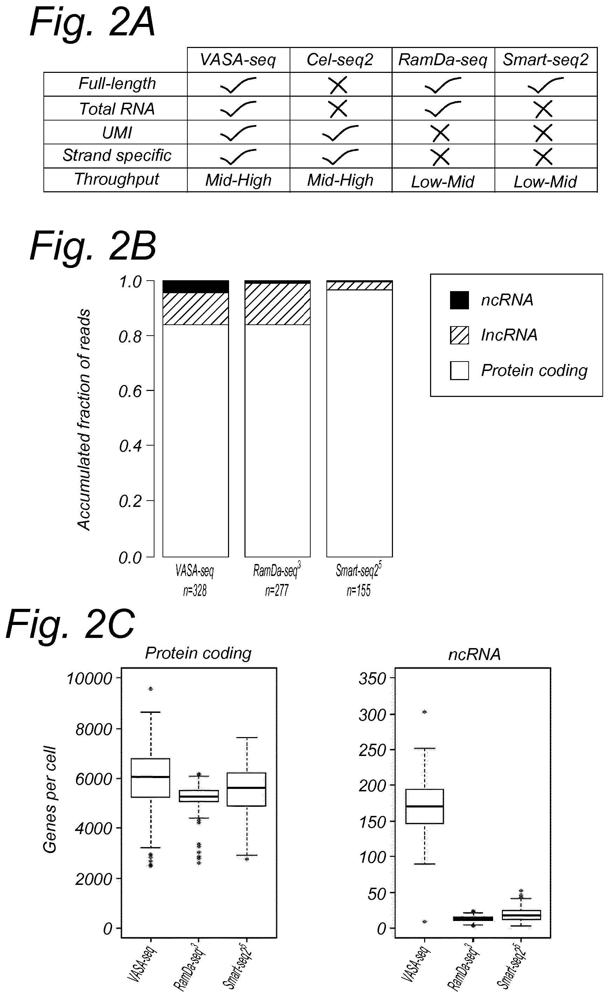

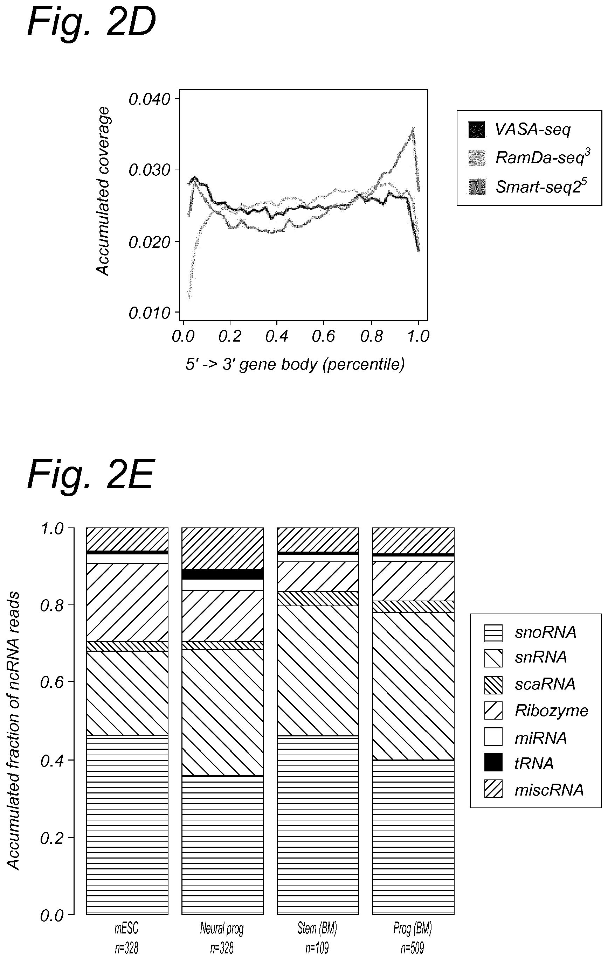

**Figure 2****:** comparison of the VASA-seq methods to other single cell sequencing methods. A) schematic representation of the advantages of VASA-seq compared to the known methods RamDa-seq, Smart-seq2 and Cel-Seq. B) detected RNA species in mouse embryonic stem cells (mESC). C) Number of detected genes in mESC (~130,000 read per cell) D) Gene body coverage E) Detected ncRNAs with VASAseq.

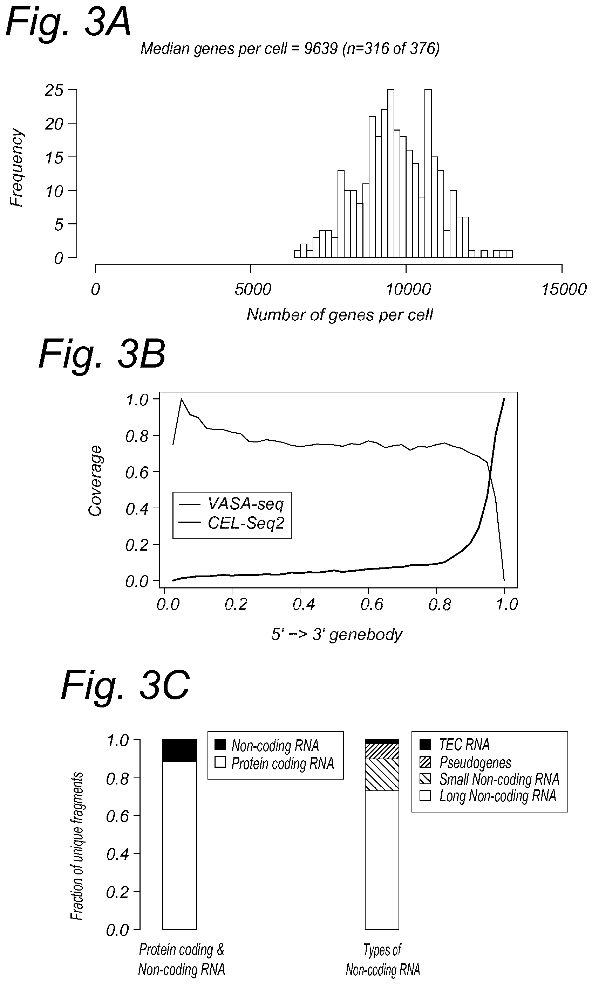

**Figure 3:** A) 376 cultured mouse embryonic stem cells sorted into 384-plates containing mineral oil and barcoded primers (one barcode per well). Plates were stored at -80°C before processing with VASA-seq. Final DNA libraries were sequenced to a depth of ~85 million reads on the Illumina NextSeq instrument. Data was mapped with STAR and unique fragments and genes were obtained. Histogram shows the number of genes detected per cell. Low quality cells were filtered away, 316 out of the 376 were kept. B) Average coverage across the gene body of all protein coding genes. A flat line, as mostly seen for VASA-seq indicates even coverage across the whole transcript from 5'-to 3'-end. Even coverage is needed for full-length detection of transcripts. VASA-seq exhibit a slight bias at the 5'-end but overall shows even coverage. Data from CEL-Seq2 (K562 cells) are used as reference for a method with clear 3'-end bias. C) Barplots showing detection of different RNA species (rRNA excluded). Left plot shows that approximately 10% of the detected fragments in VASA-seq are derived from Non-coding RNAs. This does not correspond to unique molecules as protein coding genes are for example around 10x longer than small Non-coding RNAs. Right plot shows the fractions of different types of Non-coding RNA, out of the 10% shown in the left plot.

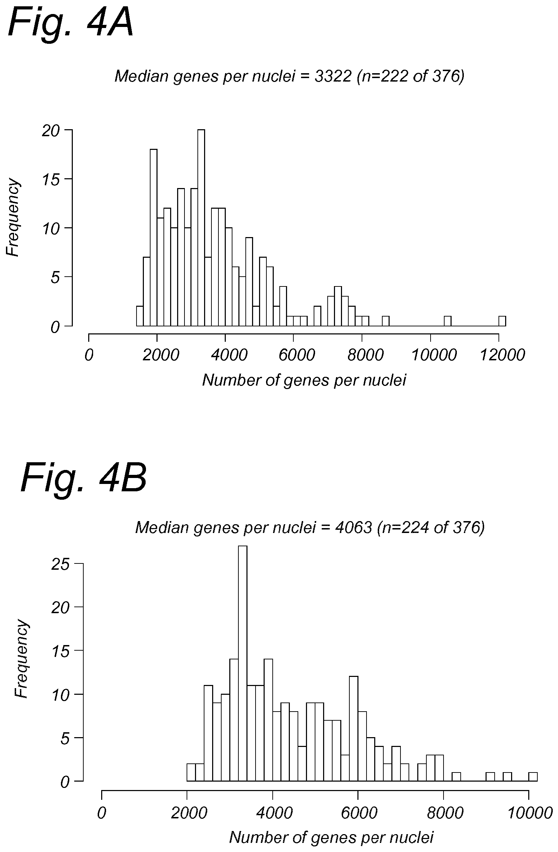

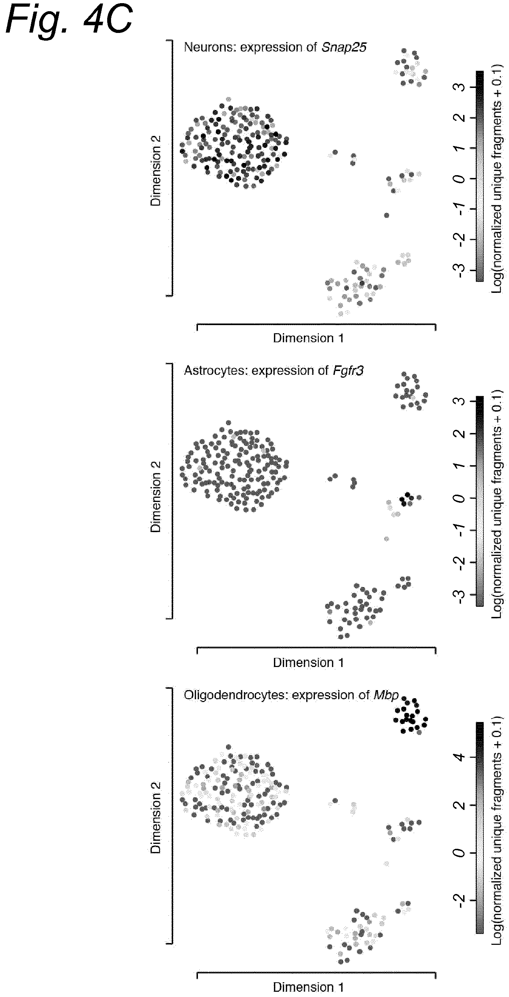

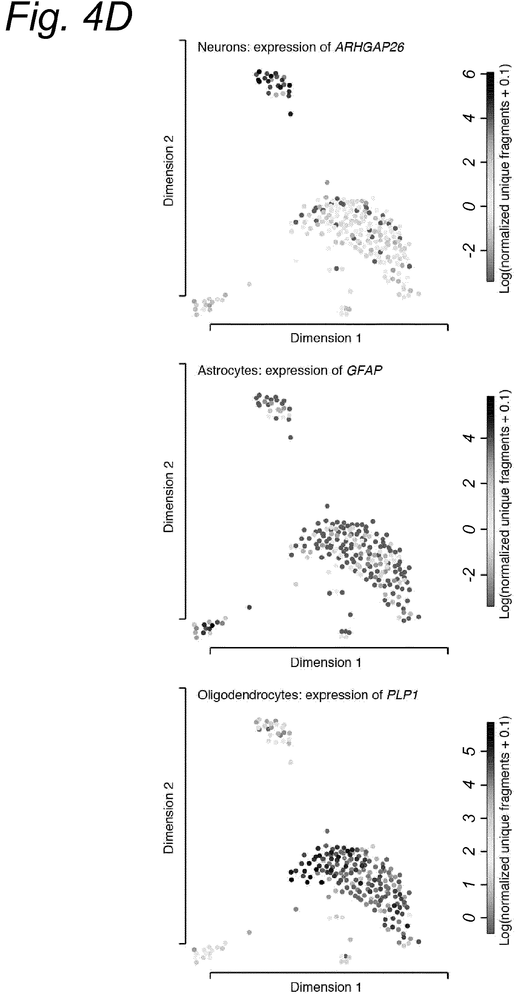

**Figure 4****:** A) 376 nuclei, dissociated from whole mouse brain, were sorted into 384-plates containing mineral oil and barcoded primers (one barcode per well). Plates were stored at -80°C before processing with VASA-seq. Final DNA libraries were sequenced to a depth of ~48 million reads on the Illumina NextSeq instrument. Data was mapped with STAR and unique fragments and genes were obtained. Histogram shows the number of genes detected per nuclei. Low quality nuclei were filtered away, 222 out of the 376 were kept. B) 376 nuclei, dissociated from the subventricular zone of a post mortem human brain sample, were sorted into 384-plates containing mineral oil and barcoded primers (one barcode per well). Plates were stored at -80°C before processing with VASA-seq. Final DNA libraries were sequenced to a depth of ~59 million reads on the Illumina NextSeq instrument. Data was mapped with STAR and unique fragments and genes were obtained. Histogram shows the number of genes detected per nuclei. Low quality nuclei were filtered away, 211 out of the 376 were kept. C) Mouse nuclei were separated in t-SNE space based on total gene expression. Each dot represents one nucleus. Several clusters/groups, representing different cells types, can be seen in the plot. Known markers for Neurons (top), Astrocytes (middle) and Oligodendrocytes (bottom) are visualized, red color indicates high expression of selected genes and blue color low expression. D) Human nuclei were separated in t-SNE space based on total gene expression. Each dot represents one nucleus. Several clusters/groups, representing different cells types, can be seen in the plot. Known markers for Neurons (top), Astrocytes (middle) and Oligodendrocytes (bottom) are visualized, red color indicates high expression of selected genes and blue color low expression.

### Examples

### Material and Methods

### Part 1:

**1. Sorting and Lysis**
- Sort cells in 384-plates with mineral oil and Cel-seq2 primers.
- Spin plates for 2 min at 4°C (2000 rcf).
- Lyse cells at 65°C for 5 minutes. Spin down and cool on ice.

**2. Fragmentation**
- Dispense 50nl of the following Fragmentation mix (add **26µl** per strip tube):

| | **1x in nl** | **4 plates in µl** |
|---|---|---|
| ERCC Spike in 1:2.500 (dilute 1:50 from 1:50) | 1 | 4.5 |
| dNTP 100mM (25mM each) | 1 | 4.5 |
| Invitrogen 5x FS buffer | 48 | 221 |

- Spin plate for 2 min at 4°C (2000 rcf).
- Fragmentize RNA at 85°C for 5 minutes. Spin down and cool on ice.
**3. End repair**
- Dispense 150nl of the following End repair mix (add **44µl** per strip tube):

| **Reagent** | **1x in nl** | **4 plates in µl** |
|---|---|---|
| H₂0 | 112.5 | 270 |
| NEB T4 PNK | 12.5 | 30 |
| Invitrogen RNaseOUT | 12.5 | 30 |
| Invitrogen DTT (0.1M) | 12.5 | 30 |

- Spin plate for 2 min at 4°C (2000 rcf)
- Incubate at 37°C for 40 minutes. Spin down and cool on ice.
**4. Poly-A tailing**
- Dispense 50nl of the following mix (add **26µl** per strip tube):

| **Reagent** | **1x in nl** | **4 plates in µl** |
|---|---|---|
| Invitrogen 5x FS buffer | 23 | 106 |
| Poly (A) Polymerase | 1.5 | 7 |
| ATP (10mM-20% NH2 ATP) | 15 | 69 |
| H₂0 | 10.5 | 48 |

- Spin plate for 2 min at 4°C (2000 rcf).
- Incubate at 37°C for 15 min. Spin down and cool on ice
**5. cDNA synthesis/Reverse transcription (RT)**
- Dispense 50nl of the following mix to two plates (add **26µl** per strip tube):

| **Reagent** | **1x in nl** | **4 plates in µl** |
|---|---|---|
| Invitrogen DTT (0.1M) | 5 | 23 |
| Invitrogen SS III | 17.5 | 81 |
| H₂0 | 27.5 | 126 |

- Spin plate for 2 min at 4°C (2000 rcf).
- Incubate at 50°C for 1h.
- Spin down and cool on ice.
**6. Second strand synthesis**
- Add 1920nl (dispense 4x480nl, place on ice after each dispension) of the following second strand mix (add **120µl** per strip tube, 4x strips):

| **Reagent** | **1x in nl** | **4 plates in µl** |
|---|---|---|
| H₂0 | 1347.5 | 2960 |
| Invitrogen 2^{nd} strand buffer | 437.5 | 960 |
| dNTP 10mM | 43.75 | 96 |
| Invitrogen E.coli ligase | 15.75 | 34.8 |
| Invitrogen E.coli DNA polymerase I | 61.25 | 134.4 |
| Invitrogen RNAseH | 15.75 | 34.8 |

- Spin plate for 2 min at 4°C (2000 rcf)
- Incubate at 16 °C for 2 hours.
- Move plates to ice.
- Set a thermocycler to 75 °C.
- Incubate plates at 75°C for 20 min.
- Make plastic boxes and spin down the plates (1 min at 300rcf).
- Add 2x1000 µl mineral oil and collect all liquid "bubbles" in one 2ml Eppendorf tube per sample.
- Spin down the tubes to separate the oil and aRNA. Then pipette all aRNA into a new 2ml eppendorf tube, make sure not to get oil (the whole volume is 800 µl).
**7. Pool&Cleanup**
- Warm AMPure XP beads to room temperature.
- Add 1x volume (approx. 800µl) of mixed Ampure XP beads with bead binding buffer (diluted 1:8).
- Incubate 15 min at RT.
- Incubate on magnet stand for 5 min or until liquid is clear. Twist to get better "bands".
- Remove supernatant without disturbing the beads.
- Wash pellet carefully with 1000µl 80% ETOH. Incubate at least 30 seconds.
- Repeat above step.
- Remove as much ETOH as possible.
- Dry at RT for approx. 10 minutes or until dry.
- Elute RNA from beads with 6.4µl water, start with one tube and use the material to elute from the other.

**8. IVT**
- Add 9.6 µl of IVT mix per sample as in CEL-Seq2. Mix well and transfer everything to a 0.5 ml tube.

| **Reagent** | **1x reaction** | **4+1x reactions** |
|---|---|---|
| Ambion T7 buffer | 1.6µl | 8µl |
| Ambion T7 enzyme | 1.6µl | 8µl |
| Ambion ATP | 1.6µl | 8µl |
| Ambion CTP | 1.6µl | 8µl |
| Ambion GTP | 1.6µl | 8µl |
| Ambion UTP | 1.6µl | 8µl |

- Incubate at 37°C for 14 hours, with lid at 70°C. Set cycler to go to 4°C at end of incubation.
**9. DNA cleanup**
EXO-SAP (to remove primers):
- Add 6µl EXO-SAP to all tubes.
- Incubate at 15 minutes at 37°C, cool on ice.

**10. Pool&Cleanup**
- Warm AMPure XP beads to room temperature.
- Add 40µl of mixed Ampure XP beads to 1.5ml tubes.
- Transfer samples (22µl) to 1.5 ml eppendorf tubes.
- Incubate 15 min at RT.
- Incubate on magnet stand for 5 min or until liquid is clear.
- Remove supernatant without disturbing the beads.
- Wash pellet carefully with 1000µl 80% ETOH. Incubate at least 30 seconds.
- Repeat above step.
- Remove as much ETOH as possible.
- Dry at RT for approx. 15 minutes or until dry.
- Elute RNA from beads with 24µl water.
- Incubate away from magnet for at least 2 min.
- Incubate on magnet stand for 5 min or until liquid is clear.
- Transfer liquid to new tubes.
- Dilute samples 1:50 and run the Bioanalyzer RNA Pico total RNA. Also measure concentration with a Qubit.
- Then dilute samples to ~100/µl.

### Part 2:

**1. rRNA depletion**
**Make Hyb-mix (4+1 reactions)**

| **Reagent** | **Volume** |
|---|---|
| Hyb-buffer | 10µl |
| rRNA-dep-oligos (25µM) | 10µl |

- Add 4µl mix to 6µl of aRNA.
- Spin down and cool on ice.
- Incubate at 95°C for 2 minutes, then decrease the temperature to 45°C at a rate of 0.1°C/s.

**Make RNase-mix (4+1 reactions)**

| **Reagent** | **Volume** |
|---|---|
| Epicentre RNaseH (Thermostable) | 10µl |
| RNase buffer | 40µl |

- Add 10µl of RNase-mix to the Hyb-mix while keeping it on 45°C.
- Incubate at 45°C for 30 minutes.
- Spin down and cool on ice.

**Make DNase-mix (4+1 reactions)**

| **Reagent** | **Volume** |
|---|---|
| Promega DNase | 10µl |
| Promega 10xBuffer | 11µl |

- Add 4.2µl of DNase-mix to the reaction.
- Incubate at 37°C for 30 minutes.
- Spin down and cool on ice.

**2. Pool&Cleanup**
- Warm AMPure XP beads to room temperature.
- Add44µl of Ampure XP beads to 1.5ml tubes.
- Transfer samples (24.2µl) to 1.5 ml eppendorf tubes.
- Incubate 15 min at RT.
- Incubate on magnet stand for 5 min or until liquid is clear.
- Remove supernatant without disturbing the beads.
- Wash pellet carefully with 1000µl 80% ETOH. Incubate at least 30 seconds.
- Repeat above step.
- Remove as much ETOH as possible.
- Dry at RT for approx. 10 minutes or until dry.
- Elute RNA from beads with 6µl water.
- Incubate away from magnet for at least 2 min.
- Incubate on magnet stand for 5 min or until liquid is clear.
- Transfer liquid to new tubes.

3. **ADAPTER LIGATION**
- Set a thermo cycler to 70°C **(with heated lid at 105°C)** and prepare the four 0.2ml low binding tubes with the following, mix well by pipetting (keep cold):

| | | |
|---|---|---|
| a. | rRNA depleted aRNA | 5µl |
| a. | 3'_adapter (20µM) | 1µl |

- Heat adapter-sample-mix at 70°C for 2 min and then directly put on ice.
- Set a thermo cycler to 25°C (with lid at **25°C** or **without** the lid closed) and prepare a 1.5ml eppendorf tube with the following reagents, mix well by pipetting (keep cold):

| | | |
|---|---|---|
| i. | NEB 10x T4 RNA Ligase Reaction Buffer | 4.5µl |
| ii. | NEB T4 RNA Ligase 2, truncated | 4.5µl |
| iii. | Invitrogen RNaseOUT | 4.5µl |
| iv. | H₂O | 4.5µl |

- Add 4µl to each of the four 0.2ml tubes, mix well by pipetting (keep cold).
- Spin down. Incubate at 25°C for 1h followed by 4°C for ∞ (thermo cycler with lid at **25°C** or **without** the lid closed).
**4. 2^{nd} cDNA SYNTHESIS**
- Set a thermo cycler to 65°C (**with heated lid at 105°C**) and prepare the four 0.2ml low binding tubes with the following, mix well by pipetting (keep cold):

| | | |
|---|---|---|
| i. | Adapter ligated RNA | 10µl |
| ii. | dNTP Mix (10mM each) | 1µl |
| iii. | RT primer (20µM) | 2µl |

- Heat at 65°C for 5 min and then directly put on ice.
- Mix the following in a 1.5ml Eppendorf tube, mix well by pipetting (keep cold):

| | | |
|---|---|---|
| v. | Invitrogen 5x FS Buffer | 18µl |
| vi. | H₂O | 4.5µl |
| vii. | Invitrogen 0.1M DTT | 4.5µl |
| viii. | Invitrogen RNaseOUT (40U/µl) | 4.5µl |
| ix. | Invitrogen SS III (200u/µl) | 4.5µl |

- Add 8µl to each of the four 0.2ml tubes, mix well by pipetting (keep cold).
- Spin down. Incubate at 50°C for 1h followed by 70 °C for 15 min, 4°C for ∞ (thermo cycler **with heated lid at 85°C**).
**5. Strand degradation**
- Add 1µl RNaseA (Thermo) to each tube.
- Incubate at 37°C for 30min, 4°C for ∞ (thermo cycler **with heated lid at 70°C**).
- Do bead cleanups as previously described but use 1:1 beads (22µl) and elute in 20µl water.
- Save first supernatant and mix with (22µl) and elute in 20µl water.

**6. PCR amplification:**
- Mix the following in a tube

| | | |
|---|---|---|
| • | H₂0 | 55µl |
| • | 2x NEBNext PCR mix | 125ul |
| • | RNA PCR Primer (RP1, from Illumina kit) | 10µl |

Add to each tube:

| | | |
|---|---|---|
| • | Above mix | 38µl |
| • | Index (one per sample) | 2µl |
| • | Purified RT material | 10µl |

Amplify the tube in the thermal cycler using the following PCR cycling conditions:
- 30 seconds at 98°C
- 7-9 cycles of:
   ▪ 10 seconds at 98°C
   ▪ 30 seconds at 60°C
   ▪ 30 seconds at 72°C
- 10 minutes at 72°C
- Hold at 4°C

7. **Size selection**
- Add 50µl water to each sample.
- Add 50µl bead to each sample, mix by pipetting (~10 times).
- Bind for 5 min, then place on magnet.
- When clear, take supernatant **and transfer to a new tube,** then add 30µl beads, mix by pipetting (~10 times). The old beads you can throw away.
- Bind for 10 min, then place on magnet.
- When clear, remove supernatant wash beads with 1000µl 80% EtOH.
- Repeat the wash one more time.
- Let beads dry, the elute in 25µl water.

**8. Bead Cleanup**
- Prewarm beads to room temperature.
- Vortex AMPure XP Beads until well dispersed, then add 20µl to the 25µl sample. Mix entire volume up ten times to mix thoroughly.
- Incubate at room temperature for 10 min.
- Place on magnetic stand for at least 5 min, until liquid appears clear.
- Remove and discard the supernatant.
- Add 1000µl freshly prepared 80% EtOH.
- Incubate at least 30 seconds, then remove and discard supernatant without disturbing beads.
- Add 1000µl freshly prepared 80% EtOH
- Incubate at least 30 seconds, then remove and discard supernatant without disturbing beads.
- Air dry beads for 10 min, or until completely dry.
- Resuspend with 10µl water. Pipette entire volume up and down ten times to mix thoroughly.
- Incubate at room temperature for 2 min.
- Place on magnetic stand for 5 min, until liquid appears clear.
- Run the Bioanalyzer DNA HS. Also measure concentration with a Qubit.

### Example 1

For protein coding genes, VASA-seq detected about 10% more genes at the same sequencing depth compared to RamDa-seq (Figure 2C). Compared to Cel-seq2 also and at the same sequencing depth, VASA-seq detected around 2.5x times more genes. For ncRNA (not including IncRNA), VASA-seq detects at least 10-20x more genes than RamDa-seq and Cel-seq2 (Figure 2B). IncRNA are about the same for RamDa seq and V ASA-seq.

## Claims

1. A method for preparing an sequencing library, preferably a deep-sequencing library, wherein the method comprises the steps of:
- step a) providing a sample containing RNA;
- step b) fragmenting the RNA;
- step c) end-repair to add an OH group at the 3' end of the fragmented RNA followed by polyadenylating the fragmented RNA;
- step d) hybridizing a poly-T primer to the polyadenylated RNA and performing reverse transcription of the hybridized RNA thereby obtaining cDNA;
- step e) performing a second strand synthesis;
- step f) *in vitro* transcription of the cDNA obtained in step d) thereby obtaining amplified RNA (aRNA);
- step g) ribosomal-RNA (rRNA) depletion; and
- step h) ligating an oligonucleotide adapter to the aRNA obtained in step f),
- step i) performing reverse transcription of the aRNA to obtain cDNA;
- step j) degrading the remaining aRNA;
wherein the poly-T primer comprises at least one of an identifier sequence (barcode) and a unique molecular identifier (UMI).

2. The method according to claim 1, wherein the RNA sample is a cellular RNA sample, preferably wherein the RNA sample is a single cell, more preferably wherein the RNA is from a cell nucleus, most preferably from a single cell nucleus.

3. The method according to claim 1 or 2, wherein the RNA is a small non-coding RNA, preferably selected from the group consisting of at least one of a microRNA, a snRNA and a snoRNA.

4. The method according to any one of claims 1 - 3, wherein the fragmentation is performed by exposure to a divalent metal-cation at a temperature between about 55-100°C.

5. The method according to claim 4, wherein the divalent metal cation is selected from the group consisting of Mg²⁺, Mn²⁺, Ca ²⁺ and Zn ²⁺ , preferably the divalent metal cation is Mg²⁺.

6. The method according to any one of the preceding claims, wherein the method further comprises one or more of the following steps:
- step k) optionally amplifying the cDNA to generate a cDNA library comprising double- stranded cDNA with sequencing primer binding sites;
- step I) selecting by size the cDNA library obtained in step k); and
- step m) sequencing the, optionally size selected, cDNA library from step k).

7. The method according to claim 1, wherein the oligonucleotide adapter in step h) comprises a barcode and optionally an UMI.

8. The method according to claim 6 wherein the size of the size selected PCR products is between 150bp and 1000bp, preferably the size of the selected PCR products is between 300-450.

9. The method according to any one of claims 6-8, wherein an adapter is ligated to at least one of:
- the RNA provided in the sample of step a);
- the single-stranded cDNA obtained in step d);
- the double-stranded cDNA obtained in step e);
- the aRNA obtained in step f); and
- the cDNA obtained in step i).

## Patentansprüche

1. Verfahren zur Herstellung einer Sequenzierungsbibliothek, vorzugsweise einer Tiefensequenzierungs-Bibliothek, wobei das Verfahren die folgenden Schritte umfasst:
- Schritt a) Bereitstellen einer RNA enthaltenden Probe;
- Schritt b) Fragmentieren der RNA;
- Schritt c) Endreparatur, um eine OH-Gruppe an das 3'-Ende der fragmentierten RNA anzufügen, gefolgt von Polyadenylierung der fragmentierten RNA;
- Schritt d) Hybridisieren eines Poly-T-Primers an die polyadenylierte RNA und Durchführen einer reversen Transkription der hybridisierten RNA, wodurch cDNA erhalten wird;
- Schritt e) Durchführen einer zweiten Strangsynthese;
- Schritt f) *in vitro* Transkription der in Schritt d) erhaltenen cDNA, wodurch amplifizierte RNA (aRNA) erhalten wird;
- Schritt g) ribosomale RNA (rRNA)-Depletion; und
- Schritt h) Ligieren eines Oligonukleotid-Adapters an die in Schritt f) erhaltene aRNA,
- Schritt i) Durchführen einer reversen Transkription der aRNA, um cDNA zu erhalten;
- Schritt j) Degradieren der verbleibenden aRNA;
wobei der Poly-T-Primer mindestens eine Identifikatorsequenz (Barcode) und einen eindeutigen molekularen Identifikator (UMI) umfasst.

2. Verfahren nach Anspruch 1, wobei die RNA-Probe eine zelluläre RNA-Probe ist, wobei die RNA-Probe vorzugsweise eine einzelne Zelle ist, wobei die RNA noch bevorzugter aus einem Zellkern stammt, am meisten bevorzugt aus einem einzelnen Zellkern.

3. Verfahren nach Anspruch 1 oder 2, wobei die RNA eine kleine nicht-kodierende RNA ist, vorzugsweise ausgewählt aus der Gruppe bestehend aus mindestens einem von einer microRNA, einer snRNA und einer snoRNA.

4. Verfahren nach einem der Ansprüche 1-3, wobei die Fragmentierung durch Einwirkung eines zweiwertigen Metallkations bei einer Temperatur zwischen etwa 55-100 °C durchgeführt wird.

5. Verfahren nach Anspruch 4, wobei das zweiwertige Metallkation aus der Gruppe ausgewählt ist, die aus Mg²⁺, Mn²⁺, Ca²⁺ und Zn²⁺ besteht, wobei das zweiwertige Metallkation vorzugsweise Mg²⁺ ist.

6. Verfahren nach einem der voranstehenden Ansprüche, wobei das Verfahren weiter einen oder mehrere der folgenden Schritte umfasst:
- Schritt k) optionales Amplifizieren der cDNA, um eine cDNA-Bibliothek zu erzeugen, die doppelsträngige cDNA mit Sequenzierungsprimer-Bindungsstellen umfasst;
- Schritt I) Auswählen der in Schritt k) erhaltenen cDNA-Bibliothek nach Größe; und
- Schritt m) Sequenzieren der optional nach Größe ausgewählten cDNA-Bibliothek aus Schritt k).

7. Verfahren nach Anspruch 1, wobei der Oligonukleotidadapter in Schritt h) einen Barcode und optional einen UMI umfasst.

8. Verfahren nach Anspruch 6, wobei die Größe der nach Größe ausgewählten PCR-Produkte zwischen 150 bp und 1000 bp liegt, vorzugsweise liegt die Größe der ausgewählten PCR-Produkte zwischen 300-450.

9. Verfahren nach einem der Ansprüche 6-8, wobei ein Adapter an mindestens eines der folgenden ligiert wird:
- die in der Probe aus Schritt a) bereitgestellte RNA;
- die in Schritt d) erhaltene einzelsträngige cDNA;
- die in Schritt e) erhaltene doppelsträngige cDNA;
- die in Schritt f) erhaltene aRNA; und
- die in Schritt i) erhaltene cDNA.

## Revendications

1. Procédé de préparation d'une banque de séquençage, de préférence une banque de séquençage profond, où le procédé comprend les étapes suivantes :
- étape a) fourniture d'un échantillon contenant de l'ARN ;
- étape b) fragmentation de l'ARN ;
- étape c) réparation d'extrémité pour ajouter un groupe OH à l'extrémité 3' de l'ARN fragmenté, puis polyadénylation de l'ARN fragmenté ;
- étape d) hybridation d'une amorce poly-T à l'ARN polyadénylé et transcription inverse de l'ARN hybridé, obtenant ainsi de l'ADNc ;
- étape e) synthèse du second brin ;
- étape f) transcription *in vitro* de l'ADNc obtenu à l'étape d), obtenant ainsi de l'ARN amplifié (ARNa) ;
- étape g) déplétion de l'ARN ribosomique (ARNr) ; et
- étape h) ligature d'un adaptateur oligonucléotidique à l'ARNa obtenu à l'étape f),
- étape i) transcription inverse de l'ARNa pour obtenir de l'ADNc ;
- étape j) dégradation de l'ARNa restant ;
où l'amorce poly-T comprend au moins un parmi une séquence d'identification (code-barres) et un identifiant moléculaire unique (UMI).

2. Procédé selon la revendication 1, où l'échantillon d'ARN est un échantillon d'ARN cellulaire, de préférence où l'échantillon d'ARN est une cellule unique, plus préférablement où l'ARN provient d'un noyau cellulaire, préférentiellement d'un noyau d'une cellule unique.

3. Procédé selon la revendication 1 ou 2, où l'ARN est un petit ARN non codant, de préférence choisi du groupe constitué par au moins un parmi un microARN, un snARN et un snoARN.

4. Procédé selon l'une quelconque des revendications 1 à 3, où la fragmentation est réalisée par exposition à un cation métallique divalent à une température comprise entre environ 55 et 100 °C.

5. Procédé selon la revendication 4, où le cation métallique divalent est choisi du groupe constitué par Mg²⁺, Mn²⁺, Ca²⁺ et Zn²⁺, de préférence le cation métallique divalent est Mg^{2+.}

6. Procédé selon l'une quelconque des revendications précédentes, où le procédé comprend en outre une ou plusieurs des étapes suivantes :
- étape k) amplification facultative de l'ADNc pour générer une banque d'ADNc comprenant de l'ADNc double brin avec des sites de liaison aux amorces de séquençage;
- étape l) sélection par taille de la banque d'ADNc obtenue à l'étape k) ; et
- étape m) séquençage de la banque d'ADNc, facultativement sélectionnée en fonction de la taille, obtenue à l'étape k).

7. Procédé selon la revendication 1, où l'adaptateur oligonucléotidique de l'étape h) comprend un code-barres et éventuellement un UMI.

8. Procédé selon la revendication 6, où la taille des produits de PCR sélectionnés en fonction de la taille est comprise entre 150 pb et 1000 pb, de préférence la taille des produits de PCR sélectionnés est comprise entre 300 et 450 pb.

9. Procédé selon l'une quelconque des revendications 6 à 8, où un adaptateur est ligaturé à au moins l'un parmi :
- l'ARN fourni dans l'échantillon de l'étape a) ;
- l'ADNc simple brin obtenu à l'étape d) ;
- l'ADNc double brin obtenu à l'étape e) ;
- l'ARNa obtenu à l'étape f) ; et
- l'ADNc obtenu à l'étape i).
